# EUROPEAN PATENT SPECIFICATION

(11) **EP 1 812 600 B1**
(45) Date of publication and mention of the grant of the patent: **23.12.2009**
(21) Application number: 05817084.6
(22) Date of filing: 02.11.2005
(51) Int. Cl.: C12P 19/34, C12Q 1/68

(54) **METHODS FOR DETECTION OF MICRORNA MOLECULES**
VERFAHREN ZUM NACHWEIS VON MIKRO-RNA-MOLEKÜLEN
PROCEDES DE DETECTION DE MOLECULES DE MICRO-ARN

(30) Priority: 02.11.2004 US 979052
(43) Date of publication of application: 01.08.2007
(73) Proprietor: Genisphere Inc., Hatfield, PA 19440 (US)
(72) Inventor: GETTS, Robert, C., Collegeville, PA 19426 (US); KADUSHIN, James, Gilbertsville, PA 19525 (US); BOWERS, Jessica, Harleysville, PA 19438 (US)
(74) Representative: Schmidt, Karsten
(86) International application number: PCT/US2005/039660
(87) International publication number: WO 2006/050433

(56) References cited:
- US-B1- 6 168 948
- US-B1- 6 344 316
- STEARS ET AL.: 'A novel, sensitive detection system for high-density microarrays using dendrimer technology' PHYSIOLOGICAL GENOMICS vol. 3, 2000, pages 93 - 99
- BARTEL D.P.: 'MicroRNAs: Genomics, Biogenesis, Mechanism and Function' CELL vol. 116, 2004, pages 281 - 297

### SEQUENCE LISTING

<110> Datascope Investment Corp.
<120> METHODS FOR DETECTION OF MICRORNA MOLECULES
<130> DSC0001-00WO
<150> 10/979,052
   <151> 2004-11-02
<160> 8
<170> PatentIn version 3.3
<210> 1
   <211> 31
   <212> DNA
   <213> Artificial
<220>
   <223> cy3 sense Strand oligonucleotide
<400> 1
   ttctcgtgtt ccgtttgtac tctaaggtgg a 31
<210> 2
   <211> 19
   <212> DNA
   <213> Artificial
<220>
   <223> Cy3 Antisense Strand oligonucleotide
<400> 2
   acacgagaat ttttttttt 19
<210> 3
   <211> 31
   <212> DNA
   <213> Artificial
<220>
   <223> cy5 sense Strand oligonucleotide
<400> 3
   attgccttgt aagcgatgtg attctattgg a 31
<210> 4
   <211> 19
   <212> DNA
   <213> Artificial
<220>
   <223> Cy5 Antisense strand oligonucleotide
<400> 4
   caaggcaatt ttttttttt 19
<210> 5
   <211> 38
   <212> DNA
   <213> Artificial
<220>
   <223> Cy3 Dendrimer oligonucleotide
<400> 5
   tccaccttag agtacaaacg gaacacgaga atttttcg 38
<210> 6
   <211> 38
   <212> DNA
   <213> Artificial
<220>
   <223> Cy5 Dendrimer oligonucleotide
<400> 6
   tccaatagaa tcacatcgct tacaaggcaa ttttttcg 38
<210> 7
   <211> 48
   <212> DNA
   <213> Artificial
<220>
   <223> Cy3 Reverse Transcription Primer
<400> 7
   ttctcgtgtt ccgtttgtac tctaaggtgg attttttttt tttttttt 48
<210> 8
   <211> 48
   <212> DNA
   <213> Artificial
<220>
   <223> Cy5 Reverse Transcription Primer
<400> 8
   attgccttgt aagcgatgtg attctattgg attttttttt tttttttt 48

## Description

### PRIORITY

This application claims priority to U.S. Patent Application No. 10/979,052 filed November 2, 2004, published under U.S. Patent Publication No. 2006/00 94 025.

### BACKGROUND ART

Recently, a class of small non-coding RNAs, termed microRNAs (miRNAs), has been identified that function in post-transcriptional regulation of gene expression in plants and amimals (Carrington and Ambrose, Science 301:336 (2003)). Originally identified in *C. elegans,* miRNAs act by basepairing to complementary sites in the 3' untranslated region (UTR) or coding sequences of their target mRNAs and repressing their translation (Wang et al., Nucleic. Acids Res. 32:1688 (2004)).

While mature miRNAs are only ∼22 nucleotides (nt) in length, they originate from hairpin regions of ∼70mer precursor (pre-miRNA) sequences through the action of Dicer complex (Lee et al., EMBO J. 21:4663 (2002)). The mature miRNA is then incorporated into the miRNP, the ribonucleoprotein complex that mediates miRNA's effects on gene regulation (Mourelatos et al., Genes Dev. 16:720 (2002)).

Bioinformatics studies predict that there are ∼100 miRNAs encoded in the worm and fly genomes, and ∼250 miRNAs encoded in the vertebrate genomes. (Lai et al., Genome Biol. 4:R42 (2003); Lim et al., Genes Dev. 17:991 (2003) ; Lim et al., Science 299:1540 (2003)) This accounts for ∼0.5-1% of the number of predicted protein-coding genes for each genome, underlining the importance of miRNAs as a class of regulatory gene products (Brennecke and Cohen, Genome Biol. 4:228 (2003)).

miRNAs have been implicated in a variety of biological processes, including flower and leaf development in plants, larval development in worms, apoptosis and fat metabolism in flies, and hematopoietic differentiation and neuronal development in mammals (Bartel, Cell 116:281 (2004)). In addition, many miRNA genes map to chromosomal regions in humans associated with cancer (e.g., fragile sites, breakpoints, regions of loss of heterozygosity, regions of amplification) (Calin et al., Proc. Natl. Acad. Sci. USA 101:2999 (2004)). Various miRNAs have also been shown to interact with the fragile X mental retardation protein (FMRP) in vivo (Jin et al., Nat. Neurosci. 7:113 (2004)), suggesting a role for these tiny RNAs in human health and disease.

Because different cell types and disease states are associated with expression of certain miRNAs, it is important to obtain both temporal and spatial expression profiles for miRNAs. Northern hybridization has been used to determine the expression levels of miRNAs (see, e.g., Sempere et al., Genome Biol. 5:R13 (2004); Aravin et al., Dev. Cell 5:337 (2003); Grad et al., Mol. Cell 11;1253 (2003); Lim et al., Genes & Dev. 17:991 (2003)), but this method is too labor intensive for high-throughput analyses. PCR-based methods have been used to monitor the expression of miRNAs, but these methods either require the use of costly gene-specific primers (see, e.g., Schmittgen et al., Nucleic Acids Res. 32:e43 (2004)) or inefficient blunt-end ligations to attach primer-binding linkers to the miRNA molecules (see, e.g., Miska et al., Genome Biol. 5:R68 (2004); Grad et al., Mol. Cell 11;1253 (2003); Lim et al., Genes & Dev. 17:991 (2003)). In addition, PCR can introduce significant biases into the population of amplified target miRNA molecules.

High-throughput microarrays have recently been developed to identify expression patterns for miRNAs in a variety of tissue and cell types (see, e.g., Babak et al., RNA 10:1813 (2004); Calin et al., Proc. Natl. Acad. Sci. USA 101:11755 (2004); Liu et al., Proc. Natl. Acad. Sci. USA 101:9740 (2004); Miska et al., Genome Biol. 5:R68 (2004); Sioud and Røsok, BioTechniques 37:574 (2004); Krichevsky et al., RNA 9:1274 (2003)). The use of microarrays has several advantages for detection of miRNA expression, including the ability to determine expression of multiple genes in the same sample at a single time point, a need for only small amounts of RNA, and the potential to simultaneously identify the expression of both precursor and mature miRNA molecules.

However, since mature miRNAs are only ∼22 nt in length and present in very limited quantities in any given tissue, these small RNAs present challenges for microarray labeling and detection (Sioud and *Røsok, BioTechniques 37:574 (2004)). For example, covalent attachment of fluorophores can be used to directly label miRNA molecules for use in microarray analyses (see, e.g., Babak et al., RNA 10:1813 (2004); MICROMAX ASAP miRNA Chemical Labeling Kit, Perkin Elmer, Shelton, CT; Label IT^{®} µArray Labeling Kit, Mirus Bio Corp., Madison, WI), but this method lacks the sensitivity to detect rare target miRNA molecules. Direct labeling can also result in intermolecular quenching of the randomly incorporated fluorophores, resulting in further decreased sensitivity. Random primed-reverse transcription of miRNA molecules has been used to produce labeled cDNA molecules for use in microarray analyses (see, e.g., Sioud and Røsok, BioTechniques 37:574 (2004); Liu et al., Proc. Natl. Acad. Sci. USA 101:9740 (2004)), but this method does not yield an accurate representation of the original full-length miRNA population. As a result, there is an immediate need for sensitive and efficient methods for labeling and detection of miRNA molecules for use in microarray analyses.

Stears et al. (Physio/. Genomics, 3:93 (2000)) discloses a system for detecting cDNA probes bound on microarrays involving the use of fluorescent oligonucleotide dendrimers. First strand cDNA is synthesized from total RNA using oligodT RT primers incorporated with capture sequences. The capture sequence-containing cDNA is then hybridized to a DNA microarray, washed and incubated with fluorescently labeled dendrimers comprising a capture oligonucleotide complementary to the capture sequences. Any dendrimer bound through hybridization of the capture oligonucleotide to the capture sequence is then detected by scanning the microarray in a laser scanner device.

### DISCLOSURE OF THE INVENTION

Applicants have invented methods for the labeling of target miRNA molecules and cDNA molecules complementary to target miRNA molecules for use in microarray analyses, wherein a capture sequence complementary to a capture reagent sequence is attached either directly to the 3' end of the miRNA molecules or to the 5' end of cDNA molecules complementary to the miRNA molecules. A capture reagent containing a label and the capture reagent sequence is then attached to the capture sequence, creating labeled nucleic acid molecules for use in miRNA microarray analyses. Applicants have discovered that quenching can be reduced and signal intensity enhanced without the need for PCR through the use of a capture sequence and a labeled capture reagent, resulting in improved methods and reagents for miRNA microarray analyses.

One aspect of this invention is directed to a method for producing a labeled target miRNA molecule comprising:
a) providing a single stranded miRNA molecule having 5' and 3' ends;
b) attaching an oligonucleotide tail onto the 3' end of the single stranded miRNA molecule;
c) providing a partially double stranded nucleic acid sequence having a sense strand and antisense strand, wherein the sense strand comprises a capture sequence at its 3' end and the antisense strand comprises a single stranded 3' overhang comprising a sequence complementary to the oligonucleotide tail;
d) annealing the partially double stranded nucleic acid sequence to the oligonucleotide tail by complementary base pairing with the 3' overhang sequence;
e) ligating the 5' end of the sense strand of the partially double stranded nucleic acid sequence to the 3' end of the oligonucleotide tail, thereby attaching the capture sequence to the 3' end of the miRNA molecule; and
f) attaching to the miRNA molecule a capture reagent comprising a label capable of producing or emitting a detectable signal and at least one nucleic acid sequence complementary to the capture sequence,
   thereby producing a labeled target miRNA molecule.

Another aspect of this invention is directed to a method for producing a labeled cDNA molecule complementary to a target miRNA molecule comprising:
a) providing a single stranded miRNA molecule having 5' and 3' ends;
b) attaching an oligonucleotide tail onto the 3' end of the single stranded miRNA molecule;
c) annealing to the oligonucleotide tail by complementary base pairing a single stranded primer comprising a capture sequence at its 5' end and a sequence complementary to the oligonucleotide tail at its 3' end;
d) extending the single stranded primer from its 3' end with reverse transciptase, thereby producing a single stranded cDNA molecule comprising a capture sequence at its 5' end; and
e) attaching to the cDNA molecule a capture reagent comprising a label capable of producing or emitting a detectable signal and at least one nucleic acid sequence complementary to the capture sequence,
thereby producing a labeled cDNA molecule complementary to a target miRNA molecule.

In some embodiments, the capture reagent is attached to the capture sequence following hybridization of the target nucleic acid to a microarray containing at least one sense or antisense miRNA probe. In other embodiments, the capture reagent is attached to the capture sequence prior to hybridization.

Applicants have also invented methods for the detection of miRNA probes on a microarray using target miRNA molecules and cDNA molecules complementary to target miRNA molecules containing a capture sequence complementary to a capture reagent sequence. Prior to or following hybridization of the capture sequence-tagged nucleic acid molecules to the microarray, a capture reagent containing a label and the capture reagent sequence is attached to the capture sequence, allowing for the detection of miRNA sense and antisense probes on the microarray.

One aspect of this invention is directed to a method for the detection of a miRNA antisense probe on a microarray comprising:
a) contacting a microarray having thereon a probe comprising the complementary nucleotide sequence of a miRNA with a labeled target miRNA molecule produced by a method comprising:
   i) providing a single stranded miRNA molecule having 5' and 3' ends;
   ii) attaching an oligonucleotide tail onto the 3' end of the single stranded miRNA molecule;
   iii) providing a partially double stranded nucleic acid sequence having a sense strand and antisense strand, wherein the sense strand comprises a capture sequence at its 3' end and the antisense strand comprises a single stranded 3' overhang comprising a sequence complementary to the oligonucleotide tail;
   iv) annealing the double stranded nucleic acid sequence to the oligonucleotide tail by complementary base pairing with the 3' overhang sequence;
   v) ligating the 5' end of the sense strand of the partially double stranded nucleic acid sequence to the 3' end of the oligonucleotide tail, thereby attaching the capture sequence to the 3' end of the miRNA molecule; and
   vi) attaching to the miRNA molecule a capture reagent comprising a label capable of producing or emitting a detectable signal and at least one nucleic acid sequence complementary to the capture sequence, thereby producing a labeled target miRNA molecule;
b) incubating the microarray and the labeled target miRNA molecule for a time and at a temperature sufficient to enable the labeled target miRNA molecule to hybridize to the miRNA antisense probe;
c) washing the microarray to remove unhybridized labeled target mRNA; and
d) detecting the signal from the hybridized labeled target miRNA molecule,
thereby detecting a miRNA antisense probe on a microarray.

Another aspect of this invention is directed to a method for the detection of a miRNA sense probe on a microarray comprising:
a) contacting a microarray having thereon a probe comprising the nucleotide sequence of a miRNA with a labeled cDNA molecule complementary to a target miRNA molecule produced by a method comprising:
   i) providing a single stranded miRNA molecule having 5' and 3' ends;
   ii) attaching an oligonucleotide tail onto the 3' end of the single stranded miRNA molecule;
   iii) annealing to the oligonucleotide tail by base pairing a single stranded primer comprising a capture sequence at its 5' end and a sequence complementary to the oligonucleotide tail at its 3' end;
   iv) extending the single stranded primer from its 3' end with reverse transciptase, thereby producing a single stranded cDNA molecule comprising a capture sequence at its 5' end; and
   v) attaching to the cDNA molecule a capture reagent comprising a label capable of producing or emitting a detectable signal and at least one nucleic acid sequence complementary to the capture sequence, thereby producing a labeled cDNA molecule complementary to a target miRNA molecule;
b) incubating the microarray and the labeled cDNA molecule for a time and at a temperature sufficient to enable the labeled cDNA molecule to hybridize to the miRNA antisense probe;
c) washing the microarray to remove unhybridized labeled cDNA; and
d) detecting the signal from the hybridized labeled cDNA molecule,
thereby detecting a miRNA sense probe on a microarray.

In some embodiments, the capture reagent is attached to the capture sequence following hybridization of the target nucleic acid to the miRNA probes on the microarray. In other embodiments, the capture reagent is attached to the capture sequence prior to hybridization.

Applicants have also invented kits for the production of labeled target miRNA molecules and cDNA molecules complementary to target miRNA molecules for use in microarray analyses, wherein a capture sequence complementary to a capture reagent sequence is attached either directly to the 3' end of the miRNA molecules or to the 5' end of cDNA molecules complementary to the miRNA molecules.

One aspect of this invention is directed to a kit for the production labeled target miRNA molecules for use in microarray analyses comprising: a partially double stranded nucleic acid sequence having a sense strand and antisense strand, wherein the sense strand comprises a capture sequence and the antisense strand comprises a single stranded 3' overhang comprising a sequence complementary to an oligonucleotide tail; and instructional materials for producing a labeled target miRNA molecule using the partially double stranded nucleic acid sequence.

In some embodiments, the kit comprises at least one enzyme for attaching an oligonucleotide tail onto the 3' end of a single stranded target miRNA molecule, wherein the oligonucleotide tail is complementary to the single stranded 3' overhang sequence of the partially double stranded nucleic acid sequence; and at least one enzyme for attaching the 5' end of the sense strand of the partially double stranded nucleic acid sequence to the 3' end of the single stranded target miRNA molecules. In further embodiments, the kit comprises a capture reagent comprising a label capable of emitting a detectable signal and a nucleic acid sequence complementary to the capture sequence. In still further embodiments, the kit comprises components, reagents and instructional materials for use of the labeled target miRNA molecules in a microarray assay.

Another aspect of this invention is directed to a kit for the production of labeled cDNA molecules complementary to target miRNA molecules for use in microarray analyses comprising: a single stranded primer comprising a capture sequence at its 5' end and a sequence complementary to an oligonucleotide tail at its 3' end; and instructional materials for producing the labeled cDNA molecules complementary to target miRNA molecules using the single stranded primer.

In some embodiments, the kit further comprises at least one enzyme for attaching an oligonucleotide tail onto the 3' end of a single stranded cDNA molecule complementary to a target miRNA molecule, wherein the oligonucleotide tail is complementary to the 3' end of the single stranded primer. In some embodiments, the kit comprises at least one reverse transcriptase for extending the single stranded primer from its 3' end to produce a single stranded cDNA molecule complementary to a target miRNA molecule comprising a capture sequence at its 5' end. In further embodiments, the kit comprises a capture reagent comprising a label capable of emitting a detectable signal and a nucleic acid sequence complementary to the capture sequence. In still further embodiments, the kit comprises components, reagents and instructional materials for use of the labeled cDNA molecules complementary to target miRNA molecules in a microarray assay.

### BRIEF DESCRIPTION OF THE DRAWINGS

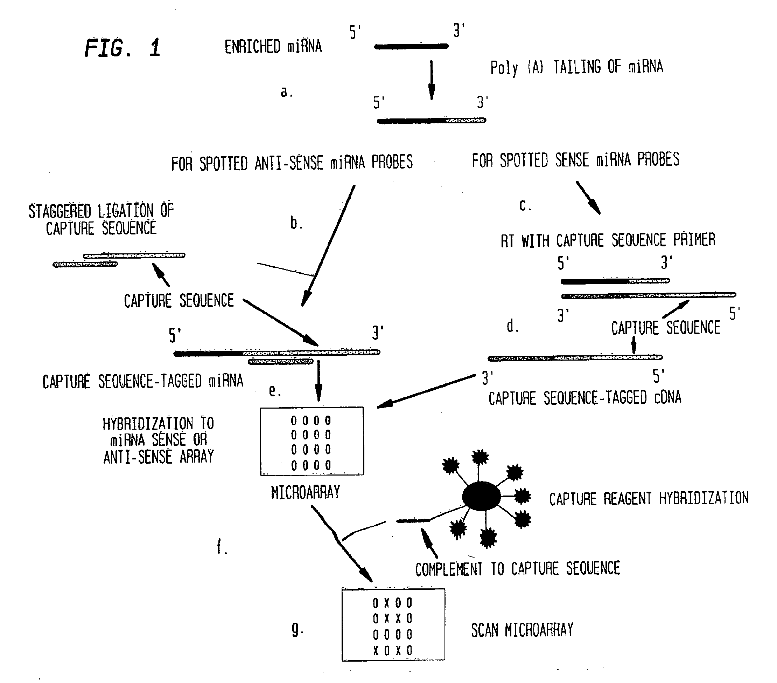

FIGS. 1a-g together depict labeling of a target mi RNA molecule or a cDNA molecule complementary to a target miRNA and the detection of sense and antisense miRNA probes according to the methods of the present invention.

### BEST MODE FOR CARRYING OUT THE INVENTION

The present invention relates to nucleic acid molecules, methods and kits for use in miRNA microarray analyses. The terms "RNA molecule", "miRNA molecule" "mRNA molecule", "DNA molecule", "cDNA molecule", and "nucleic acid molecule" are each intended to cover a single molecule, a plurality of molecules of a single species, and a plurality of molecules of different species. The term "miRNA molecule" is also intended to cover both mature and pre-miRNA molecules. Consistent with microarray terminology, "target miRNA" refers to a miRNA or complementary cDNA sequence to be labeled, while "miRNA probe" refers to an unlabeled sense or antisense miRNA sequence attached directly to a microarray support. The term "capture sequence" refers to any non-native nucleotide sequence capable of binding to a "capture reagent sequence", while the term "capture reagent" refers to a reagent containing a detectable molecule or molecules and a capture reagent sequence or sequences complementary to the capture sequence.

The methods of the present invention comprise attaching a capture sequence onto the 3' end of at least one miRNA molecule or 5' end of at least one cDNA molecule complementary to at Least one miRNA molecule; and attaching to the capture sequence a capture reagent comprising a label capable of emitting a detectable signal and a nucleotide sequence complementary to the capture sequence. The resulting labeled miRNA and cDNA molecules are then used to detect sense or antisense miRNA probes attached to microarrays, allowing miRNA expression profiles to be obtained. By using appropriately labeled target molecules and appropriately designed probes, the both mature and pre-miRNA expression profiles can be determined.

The methods of the present invention are distinct over currently available technologies that directly label target miRNA molecules by covalent attachment of fluorophores or that random prime and reverse transcribe target miRNA molecules to produce labeled cDNA molecules, both of which lack the sensitivity necessary for detecting rare target miRNA molecules following hybridization to miRNA probes. The methods of the present invention are also distinct over PCR-based labeling technologies, which can introduce amplification bias into the population of labeled target molecules.

The methods of the present invention utilize routine techniques in the field of molecular biology. Basic texts disclosing general molecular biology methods include Sambrook et al., Molecular Cloning, A Laboratory Manual (3d ed. 2001) and Ausubel et al., Current Protocols in Molecular Biology (1994).

The methods of the present invention utilize sources of RNA molecules. Preferably, the sources are enriched for miRNA molecules. Numerous methods and commercial kits are available for the enrichment of miRNA molecules from total RNA. Exampl es include the miRvana™ miRNA Isolation Kit (Ambion, Austin, TX), purification on denaturing PAGE gels (see, e.g., Miska et al., Genome Biol. 5:R68 (2004)), centrifugation with appropriately sized molecular weight cutoff filters (e.g., Microcon^{®} YM filter devices, Millipore, Billerica, MA), and sodium acetate/ethanol precipitation (see, e.g., Wang et al., Nucleic Acids Res. 32:1688 (2004)). The miRNA may be obtained from any tissue or cell source that contains miRNA, including virion, plant, and animal sources found in any biological or environmental sample. Preferably, the source is animal tissue, more preferably mammalian tissue, most preferably human tissue.

With reference to FIG. 1, a single stranded oligonucleotide tail is attached to the 3' end of the enriched single stranded miRNA molecules (see FIG. 1a). The oligonucleotide tail can be incorporated by any means that attaches nucleotides to single stranded RNA. Preferably, the oligonucleotide tail is attached to the single stranded cDNA using poly(A) polymerase (PAP), or other suitable enzyme, in the presence of appropriate nucleotides. Preferably, the oligonucleotide tail is a homopolymeric tail (i.e., polyA, polyG, polyC, or polyT). Preferably, the oligonucleotide tail is a polyA tail, generally ranging from about 3 to greater than 500 nucleotides in length, preferably from about 20 to about 100 nucleotides in length.

To produce labeled target miRNA molecules, a partially double stranded deoxynucleic acid sequence containing a sense strand capture sequence is attached to the 3' oligonucleotide tail by ligation (see FIG. 1b). This is facilitated through complementary base pairing between the 3' oligonucleotide tail and an overhang sequence at the 3' end of the antisense strand of the partially double stranded nucleic acid sequence that contains a sequence of deoxynucleotides complementary to the oligonucleotide tail. For example, if the oligonucleotide tail is a polyA tail, the 3' overhang of the partially double stranded deoxynucleic acid sequence will contain a sequence of deoxythymidines at its 3' end, generally ranging from about 3 to greater than 50 nucleotides in length, preferably from about 10 to about 30 nucleotides in length. The particular nucleotide sequence of the 3' overhang sequence does not have to be perfectly (i.e., 100%) complementary to the particular nucleotide sequence of the 3' oligonucleotide tail, nor does the length of the 3' overhang sequence need to be exactly equal to the length of the 3' oligonucleotide tail, for the sequences to be considered complementary to each other. Those of skill in the art will recognize that all that is required is that there be sufficient complementarity between the two sequences so that the 3' overhang can anneal to the 3' oligonucleotide tail, thus properly positioning the capture sequence at the 3' end of the miRNA molecule. Once properly positioned, the capture sequence is attached to the 3' oligonucleotide tail by ligation. Such overhang or "staggered" ligation reactions are more efficient and can be performed at higher temperatures than blunt-end ligation reactions. In addition, the use of an oligonucleotide tail allows for ligation of the capture sequence DNA to the DNA tail, which is more efficient than ligation of DNA directly to miRNA. Any DNA ligase can be used in the ligation reaction. Preferably, the DNA ligase is T4 DNA ligase.

Alternatively, to produce labeled cDNA molecules complementary to target miRNA molecules, a single stranded primer comprising a capture sequence at its 5' end and a sequence complementary to the oligonucleotide tail at its 3' end is annealed to the 3' oligonucleotide tail on the miRNA molecules (see FIG. 1c). For example, if.the oligonucleotide tail is a polyA tail, the 3' end of the single stranded primer will contain a sequence of deoxythymidines at its 3' end, generally ranging from about 3 to greater than 50 nucleotides in length, preferably from about 10 to about 30 nucleotides in length. Again, the particular nucleotide sequence of the 3' end of the primer does not have to be perfectly (i.e., 100%) complementary to the particular nucleotide sequence of the 3' oligonucleotide tail, nor does the length of the 3' end of the primer need to be exactly equal to the length of the 3' oligonucleotide tail, for the sequences to be considered complementary to each other. All that is required is that there be sufficient complementarity between the two sequences so that the primer anneals to the oligonucleotide tail. Once annealed, the 3' end of the primer is extended with reverse transciptase in the presence of deoxynucleotides to produce a RNA/cDNA heteroduplex containing a capture sequence at the 5' end of the cDNA molecules. Any reverse transcriptase can be used in the reverse transcription reaction, including thermostable and RNase H⁻ reverse transcriptases. Preferably, an RNase H⁻ reverse trancriptase is used.

Following the cDNA synthesis, the RNA is generally degraded prior to purification of the first strand cDNA molecules (see FIG. 1d). Any method that degrades RNA can be used, such as treatment with NaOH. Alternatively, the RNA can be left intact, with the first strand cDNA molecules purified from RNA/cDNA heteroduplexes. Numerous methods and kits exist for the purification of DNA molecules. Examples include the MinElute^{™} PCR Purification Kit (Qiagen, Valencia, CA).

The capture sequence-tagged miRNA or cDNA molecules are then contacted with a microarray containing either sense or antisense miRNA probes (see FIG. 1e). The microarray and the capture sequence-tagged nucleic acid molecules are incubated for a time and at a temperature sufficient to enable the capture sequence-tagged nucleic acid molecules to hybridize to the miRNA probes. Preferably, the microarray and the capture sequence-tagged nucleic acid molecules are incubated for about 0.5-72 hrs, preferably 18-24 hrs, at about 25-65°, preferably 45-65° C. In the case of capture sequence-tagged target miRNA molecules, the microarray will contain antisense miRNA probes, while in the case of capture sequence-tagged cDNA molecules complementary to target miRNA molecules, the microarray will contain sense miRNA probes. The probes can be designed for detection of both mature and pre-miRNA sequences, or the probes can be specific for pre-miRNA sequences. Comparison can give profiles for both the pre and mature sequences.

As used herein, "microarray" is intended to include any solid support containing nucleic acid probes, including slides, chips, membranes, beads, and microtiter plates. Methods for attaching miRNA probes to solid supports are well known to those of skill in the art (see, e.g., Babak et al., RNA 10:1813 (2004); Calin et al., Proc. Natl. Acad. Sci. USA 101:11755 (2004); Liu et al., Proc. Natl. Acad. Sci. USA 101:9740 (2004); Miska et al., Genome Biol. 5:R68 (2004); Sioud and Røsok, BioTechniques 37:574 (2004); Krichevsky et al., RNA 9:1274 (2003)). Alternatively, miRNA microarrays can be obtained commercially from, e.g., The Neuroscience Gene Expression Laboratory, W.M. Keck Center for Collaborative Neuroscience, Rutgers University, Piscataway, NJ.

Sense and antisense miRNA probes can be designed using known miRNA and pre-miRNA sequences publicly available from, e.g., The miRNA Registry, The Wellcome Trust Sanger Institute, Wellcome Trust Genome Campus, Hinxton, UK (Griffiths-Jones, Nucleic Acids. Res. 32:D109 (2004). Novel miRNA sequences can also be used to design miRNA probes and can be identified using computational methods (see, e.g., Ambros et al., Curr. Biol. 13:807 (2003); Grad et al., Mol. Cell 11;1253 (2003); Lai et al., Genome Biol. 4:R42 (2003); Lim et al., Genes & Dev. 17:991 (2003); Lim et al., Science 299:1540 (2003)) or miRNA cloning strategies (see, e.g., Wang et al., Nucleic Acids Res. 32:1688 (2004); Lagos-Quintana et al., Science 294:853 (2001); Lau et al., Science 294:858 (2001); Lee et al., Science 294:862 (2001)) well known to those skilled in the art.

Following hybridization of either the capture-sequence tagged miRNA or cDNA molecules to the sense or antisense miRNA probes on the microarray, a capture reagent containing a label capable of emitting a detectable and at least one capture reagent sequence complementary to the capture sequence is attached to the hybridized capture sequence-tagged nucleic acid molecules (see FIG. 1f). Again, the particular nucleotide sequence of the capture reagent sequence does not have to be perfectly (i.e., 100%) complementary to the particular nucleotide sequence of the capture sequence, nor does the length of the capture reagent sequence need to be exactly equal to the length of the capture sequence, for the sequences to be considered complementary to each other. All that is required is that there be sufficient complementarity between the two sequences so that the labeled capture reagent can attach to the capture sequence, thereby allowing detection of the hybridized miRNA probes. Different capture reagents containing different labels and different capture reagent sequences specific for different capture sequences can be used in the same microarray assay. Such multilabel assays reduce array costs as well as eliminate the need for normalization of array to array variation.

The label of the capture reagent comprises one or more molecules capable of emitting a detectable signal. By using a capture reagent, the signal-producing molecule or molecules can be positioned such that quenching is reduced or eliminated. Furthermore, the signal in the capture reagent can be amplified or enhanced without bias-introducing amplification of the target nucleic acid molecules themselves. Preferably, the capture reagent is a 3DNA^{™} Dendrimer Capture Reagent (Genisphere Inc., Hatfield, PA). Dendrimers are highly branched nucleic acid molecules that contain two types of single stranded hybridization "arms" on their surface for the attachment of a label and a capture sequence. Because a single dendrimer may have hundreds of arms of each type, the signal obtained upon hybridization is greatly enhanced. Signal enhancement using dendritic reagents is described in Nilsen et al., J. Theor. Biol. 187:273 (1997); Stears et al., Physiol. Genomics, 3:93 (2000); U.S. Patent Nos. 5, 175, 270, 5,484,904, 5, 487, 973, 6,072,043, 6, 110, 687, and 6,117,631; and U.S. Patent Publication No. 2002/0051981.

The signal producing molecule can be any molecule capable of emitting or producing a detectable signal. Such molecules include those that directly emit or produce a detectable signal, such as radioactive molecules, fluorescent molecules, and chemiluminescent molecules, as well as enzymes used in colorimetric assays, such as horseradish peroxidase, alkaline phosphatase, and β-galactosidase. Such molecules also include those that do not directly produce a detectable signal but which bind in systems that do, such as biotin/streptavidin and antigen/antibody. Preferably, the signal-producing molecule is one that directly emits or produces a detectable signal, more preferably a fluorophore, most preferably a Cy3 or Cy5 dye or other suitable dye, such as Alexa Fluor^{®} 555 or 647 dyes (Molecular Probes, Inc., Eugene, OR).

Although FIG. 1 depicts the attachment of the capture reagent to the capture sequence-tagged nucleic acid molecules following their hybridization to the miRNA probes on the microarray, the capture reagent can also be attached to the capture sequence-tagged nucleic acid molecules prior to their hybridization to the miRNA probes on the microarray. Such a "pre-hybridization" method may significantly reduce the time and labor required to perform the microarray assay (see U.S. Patent Publication No. 2002/0051981).

Following attachment of the capture reagent to the miRNA probes on the microarray, the microarray is washed to remove unhybridized labeled miRNA or cDNA molecules and the resulting hybridization pattern visualized by detection of the signal from the hybridized labeled nucleic acid molecules (see FIG. 1g). The specific wash and detection conditions utilized will necessarily depend on the specific signal-producing system employed and are well known to those skilled in the art (see, e.g., Babak et al., RNA 10:1813 (2004); Calin et al., Proc. Natl. Acad. Sci. USA 101:11755 (2004); Liu et al., Proc. Natl. Acad. Sci. USA 101:9740 (2004); Sioud and Røsok, BioTechniques 37:574 (2004); Krichevsky et al., RNA 9:1274 (2003); U.S. Patent Publication No. 2002/0051981).

The methods and reagents of the present invention can be conveniently packaged in kit form. Such kits can be used in various research and diagnostic applications. For example, methods and kits of the present invention can be used to facilitate a comparative analysis of expression of one or more miRNAs in different cells or tissues, different subpopulations of the same cells or tissues, different physiological states of the same cells or tissue, different developmental stages of the same cells or tissue, or different cell populations of the same tissue. Such analyses can reveal statistically significant differences in the levels of miRNA expression, which, depending on the cells or tissues analyzed, can then be used to facilitate diagnosis of various disease states, prognosis of disease progression, and identification of targets for disease treatment.

A wide variety of kits may be prepared according to the present invention. For example, a kit for the production of labeled target miRNA molecules may include a partially double stranded nucleic acid sequence having a sense strand and antisense strand, wherein the sense strand comprises a capture sequence and the antisense strand comprises a single stranded 3' overhang comprising a sequence complementary to an oligonucleotide tail; and instructional materials for producing labeled target miRNA molecules using the partially double stranded nucleic acid sequence.

A kit for the production of labeled cDNA molecules complementary to target miRNA molecules may include a single stranded primer comprising a capture sequence at its 5' end and a sequence complementary to the oligonucleotide tail at its 3' end; and instructional materials for producing labeled cDNA molecules complementary to target miRNA molecules using the single stranded primer.

While the instructional materials typically comprise written or printed materials, they are not limited to such. Any medium capable of storing such instructions and communicating them to an end user is contemplated by this invention. Such media include, but are not limited to, electronic storage media (e.g., magnetic discs, tapes, cartridges, chips), optical media (e.g., CD ROM), and the like. Such media may include addresses to internet sites that provide such instructional materials.

The kits may also include one or more of the following components or reagents for production of the labeled miRNA and cDNA molecules of the present invention: an RNase inhibitor; an enzyme for attaching an oligonucleotide tail onto single stranded miRNA molecules (e.g., poly(A) polymerase); a reverse transcriptase; an enzyme for attaching the partially double stranded nucleic acid sequence to the oligonucleotide tail (e.g., T₄ DNA ligase); and a capture reagent comprising a label capable of producing or emitting a detectable signal and a nucleic acid sequence complementary to the capture sequence. The kits may further include components and reagents and instructional materials for use of the labeled miRNA and cDNA molecules in microarray assays, including hybridization and wash solutions, incubation containers, cover slips, and various signal-detecting, signal-producing, signal-enhancing, and signal-preserving reagents. Additionally, the kits may include buffers, nucleotides, salts, RNase-free water, containers, vials, reaction tubes, and the like compatible with the production and use of the labeled nucleic acid molecules of the present invention. The components and reagents may be provided in numbered containers with suitable storage media.

Specific embodiments according to the methods of the present invention will now be described in the following examples. The examples are illustrative only, and are not intended to limit the remainder of the disclosure in any way.

### EXAMPLES

### Example 1

### Labeling of miRNA Molecules and Hybridization to Antisense miRNA Probes

### Tailing of miRNA

Briefly, up to 150 ng of rat brain miRNA purified using the miRvana™ miRNA Isolation Kit (Ambion) was adjusted to 15.5 µl with nuclease-free water and mixed with 5 µl 5X Reaction Buffer (50 mM Tris-HCl, pH 8.0, 10 mM MgCl₂), 5 µl 25 mM MnCl₂, 1 µl 10 mM ATP, and 1 µl (5 U) poly (A) polymerase. The mixture was briefly mixed, centrifuged and incubated in a 37° C heat block for 15 min.

### Ligation of miRNA

The tailed miRNA mixture was briefly centrifuged and mixed with 5 µl Cy3 or Cy5 Ligation Mix (Genisphere) in 5.5X Ligation Buffer (Roche Applied Science, Indianapolis, IN) and 2 µl T4 DNA ligase. The Cy3 Ligation Mix contained a sense strand oligonucleotide (175 ng/µl) having a sequence of 5'-PO₄-TTC TCG TGT TCC GTT TGT ACT CTA AGG TGG A-3' (SEQ ID NO: 1) and an antisense strand oligonucleotide (271 ng/µl) having a sequence of 5'-ACA CGA GAA TTT TTT TTT T-3' (SEQ ID NO:2). The Cy5 Ligation Mix contained a sense strand oligonucleotide (175 ng/µl) having a sequence of 5'-PO₄-ATT GCC TTG TAA GCG ATG TGA TTC TAT TGG A-3' (SEQ ID NO:3) and an antisense strand oligonucleotide (271 ng/µl) having a sequence of 5'-CAA GGC AAT TTT TTT TTT T-3' (SEQ ID NO:4). Each set of sense and antisense oligonucleotides was designed to form a partially double stranded nucleic acid molecule having a sense strand containing a capture sequence at its 3' end and an antisense strand containing 3' overhang sequence complementary to the poly (A) tail on the target miRNA molecules. The mixture was briefly mixed, centrifuged and incubated at room temperature for 30 min. The reaction was stopped by addition of 3.5 µl 0.5 M EDTA. The volume was adjusted to 100 µl by addition of 64.5 µl 1X TE buffer, pH 8.0, and the mixture briefly mixed and centrifuged. The tagged miRNA mixture was purified using the Qiagen MinElute^{™} PCR Purification Kit according to the manufacturer's protocol and eluted with 10 µl EB buffer.

### Tagged miRNA Microarray Hybridization

Prior to preparing microarray hybridization mixtures, the 2X SDS-based Hybridization Buffer (2X SSC, 4x Denhardt's Solution, 1% SDS, 0.5 M sodium phosphate, 2 mM EDTA, pH 8.0) and 2X Enhanced Hybridization Buffer (ExpressHyb^{™} buffer (BD Biosciences Clontech, Palo Alto, CA) diluted to 75% with nuclease-free water) were thawed and resuspended. Dual color microarray hybridization mixtures using these buffers were prepared according to the tables below:

### Option 1 (Recommended): Use of Enhanced Hybridization Buffer

| **Glass Coverslip Size (mm)** | **24x50** | **24x60** |
|---|---|---|
| **Final Hybridization Volume** | **50µl** | **60µl** |
| Cy3-tagged miRNA | 10µl | 10 µl |
| Cy5-tagged miRNA | 10µl | 10µl |
| Nuclease Free Water | 5µl | 10µl |
| 2X Enhanced Hybridization Buffer | 25µl | 30µl |

### Option 2: Use of SDS-based Hybridization Buffer

| **Glass Coverslip Size (mm)** | **24x50** | **24x60** |
|---|---|---|
| **Final Hybridization Volume 40µl** | **40µl** | **50µl** |
| Cy3-tagged miRNA | 10µl | 10µl |
| Cy5-tagged miRNA | 10µl | 10µl |
| Nuclease Free Water | 0µl | 5µl |
| 2X SDS-based Hybridization Buffer | 20µl | 25µl |

For single color assays, only one dye-tagged miRNA population is included in the chosen hybridization mixture, with the remaining volume made up with nuclease free water.

The chosen hybridization mixture was gently mixed, briefly centrifuged, and heated first at 75-80° C for 10 min and then at hybridization temperature (50-54° C) prior to microarray loading. A microarray was prepared by spotting 22-50mer antisense miRNA probes representing mature rat miRNA sequences (Compugen, Jamesburg, NJ) onto a poly-L-lysine-coated slide in 1X SSC buffer. The hybridization mixture was gently mixed, briefly centrifuged, and applied to the microarray prewarmed at 50-54° C, taking care to leave any precipitate at the bottom of hybridization mixture tube. After applying a cover slip, the microarray was incubated overnight at 50-54° C in a dark humidified glass Coplin jar.

The coverslip was removed by washing the microarray in 2X SSC, 0.2% SDS wash buffer prewarmed to 42° C. The microarray was sequentially washed in prewarmed 2X SSC, 0.2% SDS wash buffer for 15 min, 2X SSC for 10-15 min at room temperature, and 0.2X SSC for 10-15 min at room temperature. The microarray was transferred to a dry 50 mL centrifuge tube, orienting the slide so that any adhesive bar code or label was down in the tube. The tube containing the microarray was immediately centrifuged without the tube cap at 800-1000 rpm to dry the microarray. The microarray was removed from the tube, taking care not to touch the microarray surface.

### Capture Reagent Hybridization and Detection

Prior to preparing the capture reagent hybridization mixture, the 3DNA^{™} Dendrimer Capture Reagent (20 ng/µl) (Genisphere) was thawed and resuspended by vigorous vortexing and heating according to manufacturer's instructions. Each 3DNA^{™} Dendrimer Capture Reagent contains numerous conjugated Cy3 or Cy5 fluorophores and oligonucleotides having sequences complementary to the Cy3 (5' -TCC ACC TTA GAG TAC AAA CGG AAC ACG AGA ATT TTT' CG-3'; SEQ ID NO: 5) or Cy5 capture sequence (5'-TCC AAT AGA ATC ACA TCG CTT ACA AGG CAA TTT TTT Cg-3'; SEQ ID NO: 6). A dual color capture reagent hybridization mixture was prepared according to the table below:

| **Glass Coverslip Size (mm)** | **24x50** | **24x60** |
|---|---|---|
| **Final Hybridization Volume** | **40µl** | **50µl** |
| Cy3 3DNA^{™} Capture Reagent | 2.5µl | 2.5µl |
| Cy5 3DNA^{™} Capture Reagent | 2.5µl | 2.5µl |
| Nuclease Free Water | 15µl | 20µl |
| 2X SDS-based Hybridization Buffer | 20µl | 25µl |

For single color assays', only the single corresponding Capture Reagent is included in the hybridization mixture, with the remaining volume made up with nuclease free water.

The 3DNA^{™} hybridization mixture was gently mixed, briefly centrifuged, and heated first at 75-80° C for 10 min and then at 60° C prior to microarray loading. The microarray was also prewarmed at 60° C. The 3DNA^{™} hybridization mixture was gently mixed, briefly centrifuged, and applied to the pre-warmed microarray, taking care to leave any precipitate at the bottom of hybridization mixture tube. After applying a cover slip, the microarray was incubated for 3-4 hrs at 60° C in a dark humidified glass Coplin jar.

The coverslip was removed by washing the microarray in 2X SSC, 0.2% SDS wash buffer prewarmed to 55-60° C. The microarray was sequentially washed in prewarmed 2X SSC, 0.2% SDS wash buffer for 15 min, 2X SSC for 10-15 min at room temperature, and 0.2X SSC for 10-15 min at room temperature. The microarray was transferred to a dry 50 mL centrifuge tube, orienting the slide so that any adhesive bar code or label was down in the tube. The tube containing the microarray was immediately centrifuged without the tube cap at 800-1000 rpm to dry the microarray. The microarray was removed from the tube, taking care not to touch the microarray surface. DyeSaver™2 Anti-Fade Coating (Genisphere) was applied to the microarray to preserve the fluorescent signal and the array scanned using a GenePix^{®} (Axon Instruments, Union City, CA) 4000B microarray scanner with GenePix^{®} Pro 3.0 software, thereby producing an expression profile of the miRNA sequences in the original sample.

### Example 2

### Labeling of cDNA Moleculess Complementary to miRNA Molecules and Hybridization to sense miRNA Probes

### Tailing of miRNA

Briefly, up to 150 ng of rat brain miRNA purified using the miRvana™ miRNA Isolation Kit (Ambion) was adjusted to 15.5 µl with nuclease-free water and mixed with 5 µl 5X Reaction Buffer (50 mM Tris-HCl, pH 8.0, 10 mM MgCl₂), 5 µl 25 mM MnCl₂, 1 µl 10 mM ATP, and 1 µl (5 U) poly (A) polymerase. The mixture was briefly mixed, centrifuged and incubated in a 37° C heat block for 15 min.

### Reverse Transcription of Tailed miRNA

The tailed miRNA mixture was briefly centrifuged and mixed on ice with 2 µl 30 pmoles/µl Cy3 (5'-TTC TCG TGT TCC GTT TGT ACT CTA AGG TGG ATT TTT TTT TTT TTT TTT-3'; SEQ ID NO: 7) or Cy5 (5'-ATT GCC TTG TAA GCG ATG TGA TTC TAT TGG ATT TTT TTT TTT TTT TTT-3'; SEQ ID NO:8) reverse transcription (RT) primer (Genisphere). These primers contain a capture sequence at their 5' ends and a sequence of deoxythymidines complementary to the polyA tail at their 3' end. The mixture was briefly mixed, centrifuged, incubated at 65° C for 10 min, and immediately transferred to ice for 2 min. The following reagents were added on ice for a final volume of 50 µl:
10 µl 5X First Strand Buffer (Invitrogen,
Carlsbad, CA)
5 µl 0.1 M DTT
2.5 µl 10 mM dNTP mix
1 µl Superase-In^{™} RNase Inhibitor (Ambion)
2µl (200 units) Superscript^{™} II reverse transcriptase (Invitrogen)
2.5µl Nuclease Free Water

The mixture was gently mixed and incubated at 42° C for 1 hr. The reaction was stopped and the RNA degraded by addition of 8.75 µl 0.5 M NaOH/50 mM EDTA and incubating at 65° C for 15 min. The reaction was neutralized with 12.5 µl 1 M Tris, pH 8. For dual color assays, the two tagged-cDNA populations were combined into a single tube and briefly mixed and centrifuged. The tagged cDNA was concentrated to 20 µl using a Microcon^{®} YM-10 Centrifugal Filter Device (Millipore) according manufacturer's instruction.

### Tagged cDNA Microarray Hybridization

Prior to preparing microarray hybridization mixtures, the 2X SDS-based Hybridization Buffer and 2X Enhanced Hybridization Buffer were thawed and resuspended as described above. Microarray hybridization mixtures using these buffers were prepared according to the tables below:

### Option 1 (Recommended): Use of Enhanced Hybridization Buffer

| **Glass Coverslip Size, mm** | **24x50** | **24x60** |
|---|---|---|
| **Final Hybridization Volume** | **50µl** | **60µl** |
| Tagged cDNA | 20µl | 20µl |
| Nuclease Free Water | 5µl | 10µl |
| 2X Enhanced Hybridization Buffer | 25µl | 30µl |

### Option 2: Use of SDS-based Hybridization Buffer

| **Glass Coverslip Size, mm** | **24x50** | **24x60** |
|---|---|---|
| **Final Hybridization Volume** | **40µl** | **50µl** |
| Tagged cDNA | 20µl | 20µl |
| Nuclease Free Water | 0µl | 5µl |
| 2X SDS-based Hybridization Buffer | 20µl | 25µl |

For single color assays, only one dye-tagged cDNA population is included in the chosen hybridization mixture.

The chosen hybridization mixture was gently mixed, briefly centrifuged, and heated first at 75-80° C for 10 min and then at hybridization temperature (45-50° C) prior to microarray loading. A microarray was prepared by spotting 22-50mer sense miRNA probes representing mature rat miRNA sequences (Compugen) onto a poly-L-lysine-coated slide in 1X SSC buffer. The hybridization mixture was gently mixed, briefly centrifuged, and applied to the microarray prewarmed at 45-50° C, taking care to leave any precipitate at the bottom of hybridization mixture tube. After applying a cover slip, the microarray was incubated overnight at 45-50° C in a dark humidified glass Coplin jar.

The coverslip was removed by washing the microarray in 2X SSC, 0.2% SDS wash buffer prewarmed to 42° C. The microarray was sequentially washed in prewarmed 2X SSC, 0.2% SDS wash buffer for 15 min, 2X SSC for 10-15 min at room temperature, and 0.2X SSC for 10-15 min at room temperature. The microarray was transferred to a dry 50 mL centrifuge tube, orienting the slide so that any adhesive bar code or label was down in the tube. The tube containing the microarray was immediately centrifuged without the tube cap at 800-1000 rpm to dry the microarray. The microarray was removed from the tube, taking care not to touch the microarray surface.

### Capture Reagent Hybridization and Detection

Prior to preparing the capture reagent hybridization mixture, the 3DNA^{™} Dendrimer Capture Reagent (Genisphere) was thawed and resuspended as described above. A capture reagent hybridization mixture was prepared according to the table below:

| **Glass coverslip Size (mm)** | **24x50** | **24x60** |
|---|---|---|
| **Final Hybridization Volume** | **40µl** | **50µl** |
| Cy3 3DNA^{™} Capture Reagent | 2.5µl | 2.5µl |
| Cy5 3DNA^{™} Capture Reagent | 2.5µl | 2.5µl |
| Nuclease Free Water | 15µl | 20µl |
| 2X SDS-based Hybridization Buffer | 20µl | 25µl |

For single color assays, only the single corresponding Capture Reagent is included in the hybridization mixture, with the remaining volume made up with nuclease free water.

The 3DNA^{™} hybridization mixture was gently mixed, briefly centrifuged, and heated first at 75-80° C for 10 min and then at 60° C prior to microarray loading. The microarray was also prewarmed at 60° C. The 3DNA^{™} hybridization mixture was gently mixed, briefly centrifuged, and applied to the pre-warmed microarray, taking care to leave any precipitate at the bottom of hybridization mixture tube. After applying a cover slip, the microarray was incubated for 3-4 hrs at 60° C in a dark humidified glass Coplin jar.

The coverslip was removed by washing the microarray in 2X SSC, 0.2% SDS wash buffer prewarmed to 55-60° C. The microarray was sequentially washed in prewarmed 2X SSC, 0.2% SDS wash buffer for 15 min, 2X SSC for 10-15 min at room temperature, and 0.2X SSC for 10-15 min at room temperature. The microarray was transferred to a dry 50 mL centrifuge tube, orienting the slide so that any adhesive bar code or label was down in the tube. The tube containing the microarray was immediately centrifuged without the tube cap at 800-1000 rpm to dry the microarray. The microarray was removed from the tube, taking care not to touch the microarray surface. DyeSaver™2 Anti-Fade Coating (Genisphere) was applied to the microarray to preserve the fluorescent signal and the array scanned using a GenePix^{®} (Axon Instruments, Union City, CA) 4000B microarray scanner with GenePix^{®} Pro 3.0 software, thereby producing an expression profile of the miRNA sequences in the original sample.

### Example 3

### Kit for Labeling of Target miRNA Molecules Hybridization to Antisense miRNA Probes

A kit for the production and microarray hybridization of labeled target miRNA molecules was assembled with the following components:
Cy3 and Cy5 3DNA^{™} Capture Reagent (20 ng/µl) (Genisphere);
5X Reaction Buffer (50 mM Tris-HCl, pH 8.0, 10 mM MgCl₂) ;
MnCl₂ (25 mM);
ATP Mix (10 mM);
Poly(A) Polymerase (5 U/µl);
2X SDS-Based Hybridization Buffer (2X SSC, 4x Denhardt's Solution, 1% SDS, 0.5 M sodium phosphate, 2 mM EDTA, pH 8.0);
2X Enhanced Hybridization Buffer (ExpressHyb^{™} buffer (BD Biosciences Clontech) diluted to 75% with nuclease-free water);
T4 DNA Ligase (1 U/µl);
Cy3 and Cy5 Ligation Mix (75 ng/µl sense oligonucleotide and 271 ng/µl antisense oligonucleotide) (Genisphere); and
Nuclease-Free Water.

The components were placed in numbered vials and placed in a container with a printed instruction manual for the production and microarray hybridization of labeled target miRNA molecules using the kit components.

### Example 4

### Kit for Labeling of cDNA Molecules Complementary to Target miRNA Molecules and Hybridization to Sense miRNA Probes

A kit for the production and microarray hybridization of labeled cDNA molecules complementary to target miRNA molecules was assembled with the following components:
Cy3 and Cy5 3DNA^{™} Capture Reagent (20 ng/µl) (Genisphere);
5X Reaction Buffer (50 mM Tris-HCl, pH 8.0, 10 mM MgCl₂);
MnCl₂ (25 mM);
ATP Mix (10 mM);
Poly(A) Polymerase (5 U/µl);
2X SDS-Based Hybridization Buffer (2X SSC, 4x Denhardt's Solution, 1% SDS, 0.5 M sodium phosphate, 2 mM EDTA, pH 8.0);
2X Enhanced Hybridization Buffer (ExpressHyb^{™} buffer (BD Biosciences Clontech) diluted to 75% with nuclease-free water);
dNTP Mix (10 mM);
Cy3 and Cy5 RT Primers (30 pmoles/µl) (Genisphere);
Superase-In^{™} RNase Inhibitor (Ambion); and
Nuclease-Free Water.

The components were placed in numbered vials and placed in a container with a printed instruction manual for the production and microarray hybridization of labeled cDNA molecules complementary to target miRNA molecules using the kit components.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the present invention as defined by the following claims.

## Claims

1. A method for producing a labeled target miRNA molecule comprising:
a) providing a single stranded miRNA molecule having 5' and 3' ends;
b) attaching an oligonucleotide tail onto the 3' end of said single stranded miRNA molecule using poly(A) polymerase;
c) providing a partially double stranded nucleic acid sequence having a sense strand and antisense strand, wherein the sense strand comprises a capture sequence at its 3' end and the antisense strand comprises a single stranded 3' overhang comprising a sequence complementary to said oligonucleotide tail;
d) annealing said partially double stranded nucleic acid sequence to said oligonucleotide tail by complementary base pairing with the 3' overhang sequence;
e) ligating the 5' end of the sense strand of said partially double stranded nucleic acid sequence to the 3' end of said oligonucleotide tail, thereby attaching said capture sequence to the 3' end of said miRNA molecule; and
f) attaching to said miRNA molecule a capture reagent comprising a label capable of producing or emitting a detectable signal and at least one nucleic acid sequence complementary to said capture sequence,
thereby producing a labeled target miRNA molecule.

2. The method of claim 1, wherein ligation is performed using T4 DNA ligase.

3. The method of claim 1, wherein the label comprises a fluorophore.

4. A method for producing a labeled cDNA molecule complementary to a target miRNA molecule comprising:
a) providing a single stranded miRNA molecule having 5' and 3' ends;
b) attaching an oligonucleotide tail onto the 3' end of said single stranded miRNA molecule using poly(A) polymerase;
c) annealing to said oligonucleotide tail by complementary base pairing a single stranded primer comprising a capture sequence at its 5' end and a sequence complementary to said oligonucleotide tail at its 3' end;
d) extending said single stranded primer from its 3' end with reverse transciptase, thereby producing a single stranded cDNA molecule comprising a capture sequence at its 5' end; and
e) attaching to said cDNA molecule a capture reagent comprising a label capable of producing or emitting a detectable signal and at least one nucleic acid sequence complementary to said capture sequence,
thereby producing a labeled cDNA molecule complementary to a target miRNA molecule.

5. The method of claim 4, wherein the single stranded primer is extended with a RNase H⁻ reverse trancriptase.

6. The method of claim 4, wherein the label comprises a fluorophore.

7. A method for the detection of a miRNA antisense probe on a microarray comprising:
a) contacting a microarray having thereon a probe comprising the complementary nucleotide sequence of a miRNA with a labeled target miRNA molecule produced by a method comprising:
i) providing a single stranded miRNA molecule having 5' and 3' ends;
ii) attaching an oligonucleotide tail onto the 3' end of said single stranded miRNA molecule using poly(A) polymerase;
iii) providing a partially double stranded nucleic acid sequence having a sense strand and antisense strand, wherein the sense strand comprises a capture sequence at its 3' end and the antisense strand comprises a single stranded 3' overhang comprising a sequence complementary to said oligonucleotide tail;
iv) annealing said double stranded nucleic acid sequence to said oligonucleotide tail by complementary base pairing with the 3' overhang sequence;
v) ligating the 5' end of the sense strand of said partially double stranded nucleic acid sequence to s aid 3' end of the oligonucleotide tail, thereby attaching the capture sequence to the 3' end of said miRNA molecule; and
vi) attaching to said miRNA molecule a capture reagent comprising a label capable of producing or emitting a detectable signal and at least one nucleic acid sequence complementary to said capture sequence; thereby producing a labeled target miRNA molecule;
b) incubating said microarray and said labeled target miRNA molecule for a time and at a temperature sufficient to enable said labeled target miRNA molecule to hybridize to said miRNA antisense probe;
c) washing said microarray to remove unhybridized labeled target mRNA; and
d) detecting the signal from the hybridized labeled target miRNA molecule,
thereby detecting a miRNA antisense probe on a microarray.

8. The method of claim 7, wherein ligation is performed using T4 DNA ligase.

9. The method of claim 7, wherein the label comprises a fluorophore.

10. The method of claim 7, wherein the microarray is incubated with the target miRNA molecule following attachment of the capture reagent.

11. The method of claim 7, wherein the microarray is incubated with the target miRNA molecule prior to attachment of the capture reagent.

12. The method of claim 7, wherein the miRNA antisense probe on a microarray is detected by hybridization of labeled target miRNA molecules producing or emitting at least two different detectable signals.

13. A method for the detection of a miRNA sense probe on a microarray comprising:
a) contacting a microarray having thereon a probe comprising the nucleotide sequence of a mi RNA with a labeled cDNA molecule complementary to a target miRNA molecule produced by a method comprising:
i) providing a single stranded miRNA molecule having 5' and 3' ends;
ii) attaching an oligonucleotide tail onto the 3' end of said single stranded miRNA molecule using poly(A) polymerase;
iii) annealing to said oligonucleotide tail by base pairing a single stranded primer comprising a capture sequence at its 5' end and a sequence complementary to said oligonucleotide tail at its 3' end;
iv) extending said single stranded primer from its 3' end with reverse transciptase, thereby producing a single stranded cDNA molecule comprising a capture sequence at its 5' end; and
v) attaching to said cDNA molecule a capture reagent comprising a label capable of producing or emitting a detectable signal and at least one nucleic acid sequence complementary to said capture sequence, thereby producing a labeled cDNA molecule complementary to a target miRNA molecule;
b) incubating said microarray and said labeled cDNA molecule for a time and at a temperature sufficient to enable said labeled cDNA molecule to hybridize to said miRNA antisense probe;
c) washing said microarray to remove unhybridized labeled cDNA; and
d) detecting the signal from the hybridized labeled cDNA molecule,
thereby detecting a miRNA sense probe on a microarray.

14. The method of claim 13, wherein the single stranded primer is extended with a RNase H⁻ reverse trancriptase.

15. The method of claim 13, wherein the label comprises a fluorophore.

16. The method of claim 13, wherein the microarray is incubated with the cDNA molecule following attachment of the capture reagent.

17. The method of claim 13, wherein the microarray is incubated with the cDNA molecule prior to attachment of the capture reagent.

18. The method of claim 13, wherein the miRNA sense probe on a microarray is detected by hybridization of labeled cDNA molecules complementary to target miRNA molecules producing or emitting at least two different detectable signals.

## Patentansprüche

1. Verfahren zum Herstellen eines markierten Ziel-miRNA-Moleküls, umfassend:
a) Bereitstellen eines einzelsträngigen miRNA-Moleküls das 5'- und 3'-Enden hat;
b) Anheften eines Oligonucleotid-Schwanzes unter Verwendung von Poly(A)-Polymerase an das 3'-Ende des einzelstrangigen miRNA-Moleküls;
c) Bereitstellen einer teilweise doppelsträngigen Nucleinsäuresequenz, die einen Sinn-Strang (Sense-Strang) und einen Gegensinn-Strang haben, wobei der Sinn-Strang eine Fangsequenz an seinem 3'-Ende umfasst und der Gegensinn-Strang einen einzelsträngigen 3'-Überhang hat, der eine Sequenz umfasst, welche komplementär zum Oligonucleotid-Schwanz ist;
d) Annealing der teilweise doppelsträngigen Nucleinsäuresequenz mit dem Oligonucleotid-Schwanz durch komplementäre Basenpaarung mit der 3'-Überhangsequenz;
e) Ligieren des 5'-Endes des Sinn-Strangs der teilweise doppelsträngigen Nucleinsäuresequenz mit dem 3'-Ende des Oligonucleotid-Schwanzes, wodurch die Fangsequenz am 3'-Ende des miRNA-Moleküls befestigt ist; und
f) Anheften eines Fangreagens, das eine Markierung umfasst, welche ein feststellbares Signal erzeugen oder emittieren kann, und mindestens einer Nucleinsäuresequenz, die zur Fangsequenz komplementär ist, am miRNA-Molekül,
wodurch ein markiertes Ziel-miRNA-Molekül erzeugt ist.

2. Verfahren nach Anspruch 1, wobei das Ligieren unter Verwendung der T4-DNA-Ligase ausgeführt ist.

3. Verfahren nach Anspruch 1, wobei die Markierung einen Fluorophor umfasst.

4. Verfahren zum Herstellen eines markierten cDNA-Moleküls, das zu einem Ziel-miRNA-Molekül ist komplementär, umfassend:
a) Bereitstellen eines einzelsträngigen miRNA-Moleküls, das 5'- und 3'-Enden hat;
b) Anheften eines Oligonucleotid-Schwanzes am 3'-Ende des einzelsträngigen miRNA-Moleküls unter Verwendung von Poly(A)-Polymerase;
c) Annealing eines einzelsträngigen Primers, der eine Fangsequenz an seinem 5'-Ende und eine Sequenz umfasst, die zum Oligonucleotid-Schwanz an seinem 3'-Ende komplementär ist, durch komplementäre Basenpaarung am Oligonucleotid-Schwanz;
d) Erweitern des einzelsträngigen Primers von seinem 3'-Ende aus mit Umkehrtranskriptase und **dadurch** Erzeugen eines einzelsträngigen cDNA-Moleküls, das eine Fangsequenz an seinem 5'-Ende hat; und
e) Anheften eines Fangreagens, das eine Markierung umfasst, die ein nachweisbares Signal erzeugen oder emittieren kann, und mindestens einer Nucleinsäuresequenz, die komplementär zur Fangsequenz ist, am cDNA-Molekül;
wodurch ein markiertes cDNA-Molekül erzeugt ist, das zu einem Ziel-miRNA-Molekül komplementär ist.

5. Verfahren nach Anspruch 4, wobei der einzelsträngige Primer mit einer RNase-H⁻-Umkehrtranskriptase erweitert ist.

6. Verfahren nach Anspruch 4, wobei die Markierung einen Fluorophor umfasst.

7. Verfahren zum Nachweis einer miRNA-Gegensinnsonde auf einem Mikroarray, umfassend:
a) Kontaktieren eines Mikroarrays, das darauf eine Sonde hat, die die komplementäre Nucleotidsequenz eines miRNA mit einem markierten Ziel-miRNA-Molekül umfasst, das mit einem Verfahren hergestellt ist, welches folgendes umfasst:
I) Bereitstellen eines einzelstrangigen miRNA-Moleküls, das 5'- und 3' Enden hat;
II) Anheften eines Oligonucleotid-Schwanzes am 3'-Ende des einzelsträngigen miRNA-Moleküls unter Verwendung von Poly(A)-Polymerase;
III) Bereitstellen einer teilweise doppelsträngigen Nucleinsäuresequenz, die einen Sinn-Strang und Gegensinn-Strang hat, wobei der Sinn-Strang eine Fangsequenz an seinem 3'-Ende umfasst und der Gegensinn-Strang einen einzelsträngigen 3'-Überhang umfasst, der eine Sequenz umfasst, die zu dem Oligonucleotid-Schwanz komplementär ist;
IV) Annealing der doppelsträngigen Nucleinsäuresequenz mit dem Oligonucleotid-Schwanz durch komplementäre Basenpaarung mit der 3'-Überhangsequenz;
V) Ligieren des 5'-Endes des Sinn-Strangs der teilweise doppelsträngigen Nucleinsäuresequenz mit dem 3'-Ende des Oligonucleotid-Schwanzes und **dadurch** Anheften der Fangsequenz am 3'-Ende des miRNA-Moleküls; und
VI) Anheften eines Fangreagens, das eine Markierung umfasst, die ein nachweisbares Signal erzeugen oder emittieren kann, und mindestens einer Nucleinsäuresequenz, die zur Fangsequenz komplementär ist, am miRNA-Molekül und
**dadurch** Erzeugen eines markierten Ziel-miRNA-Moleküls;
b) Inkubieren des Mikroarrays des markierten Ziel-miRNA-Moleküls für eine Dauer und bei einer Temperatur, die zum Hybridisieren des markierten Ziel-miRNA-Moleküls mit der miRNA-Gegensinnsonde ausreichend sind;
c) Waschen des Mikroarrays zum Entfernen der nicht hybridisierten markierten Ziel-mRNA; und
d) Nachweisen des Signals vom hybridisierten markierten Ziel-miRNA-Moleküls,
**dadurch** Nachweisen einer miRNA-Gegensinnsonde in einem Mikroarray.

8. Verfahren nach Anspruch 7, wobei das Ligieren unter Verwendung der T4-DNA-Ligase erfolgt.

9. Verfahren nach Anspruch 7, wobei die Markierung einen Fluorophor umfasst.

10. Verfahren nach Anspruch 7, wobei das Mikroarray mit dem Ziel-miRNA-Molekül nach dem Anheften des Fangreagens inkubiert ist.

11. Verfahren nach Anspruch 7, wobei das Mikroarray vor dem Anheften des Fangreagens mit dem Ziel-miRNA-Molekül inkubiert ist.

12. Verfahren nach Anspruch 7, wobei die miRNA-Gegensinnsonde auf einem Mikroarray durch Hybridisieren der markierten Ziel-miRNA-Moleküle, die mindestens zwei verschiedene nachweisbare Signale erzeugen oder emittieren, nachgewiesen ist.

13. Verfahren zum Nachweisen einer miRNA-Sinnsonde auf einem Mikroarray, umfassend:
a) Kontaktieren eines Mikroarrays, das darauf eine Sonde hat, die die Nucleinsäuresequenz eines miRNA mit einem markierten cDNA-Moleküls umfasst, das durch ein Verfahren hergestellt ist, welches folgendes umfasst:
I) Bereitstellung eines einzelsträngigen miRNA-Moleküls, das 5'- und 3'-Enden hat;
II) Anheften eines Oligonucleotid-Schwanzes am 3'-Ende des einzelsträngigen miRNA-Moleküls unter Verwendung von Poly(A)-Polymerase;
III) Annealing eines einzelsträngigen Primers, der eine Fangsequenz an seinem 5'-Ende und eine Sequenz, die zum Oligonucleotid-Schwanz an seinem 3'-Ende komplementär ist, umfasst, durch Basenpaarung am Oligonucleotid-Schwanz;
IV) Erweitern des einzelsträngigen Primers von seinem 3'-Ende aus mit Umkehrtranskriptase, **dadurch** Erzeugen eines einzelstränqiqen cDNA-Moleküls, das eine Fangsequenz an seinem 5'-Ende umfasst; und
V) Anheften eines Fangreagens, das eine Markierung umfasst, welche ein nachweisbares Signal erzeugen oder emittieren kann, und mindestens einer Nucleinsäuresequenz, die zur Fangsequenz komplementär ist, **dadurch** Erzeugen eines markierten cDNA-Moleküls, das zu einem Ziel-miRNA-Molekül komplementär ist;
b) Inkubieren des Mikroarrays und des markierten cDNA-Moleküls für eine Dauer und bei einer Temperatur, die ausreichend sind, damit das markierte cDNA-Molekül mit der miRNA-Gegensinnsonde hybridisieren kann;
c) Waschen des Mikroarrays, um nicht hybridisierte markierte cDNA zu entfernen; und
d) Nachweisen des Signals aus dem hybridisierten markierten cDNA-Molekül,
**dadurch** Nachweisen einer miRNA-Gegensinnsonde auf dem Mikroarray.

14. Verfahren nach Anspruch 13, wobei der einzelsträngige Primer mit einer RNase-H⁻-Umkehrtranskriptase erweitert ist.

15. Verfahren nach Anspruch 13, wobei die Markierung einen Fluorophor umfasst.

16. Verfahren nach Anspruch 13, wobei das Mikroarray nach dem Anheften des Fangreagens mit dem DNA-Molekül inkubiert ist.

17. Verfahren nach Anspruch 13, wobei das Mikroarray vor dem Anheften des Fangreagens mit dem cDNA-Molekül inkubiert ist.

18. Verfahren nach Anspruch 13, wobei die miRNA-Sinnsonde auf einem Mikroarray durch Hybridisieren von markierten cDNA-Molekülen nachgewiesen sind, die zu miRNA-Molekülen komplementär sind, die mindestens zwei verschiedene nachweisbare Signale erzeugen oder emittieren.

## Revendications

1. Procédé pour produire une molécule miARN cible marquée comprenant les étapes consistant à :
a) fournir une molécule miARN à brin unique ayant des extrémités 5' et 3' ;
b) attacher une queue d'oligonucléotides sur l'extrémité 3' de ladite molécule miARN à brin unique en utilisant une poly(A)polymérase ;
c) fournir une séquence d'acide nucléique partiellement à double brin ayant un brin de détection et un brin d'anti-détection, dans lequel le brin de détection comprend une séquence d'acquisition à son extrémité 3' et le brin d'anti-détection comprend une extrémité protubérante 3' à brin unique comprenant une séquence complémentaire de ladite queue d'oligonucléotides ;
d) fusionner ladite séquence d'acide nucléique partiellement à double brin sur ladite queue d'oligonucléotides par pairage de base complémentaire avec la séquence d'extrémité protubérante 3' ;
e) lier l'extrémité 5' du brin de détection de ladite séquence d'acide nucléique partiellement à double brin à l'extrémité 3' de ladite queue d'oligonucléotides, en attachant de ce fait ladite séquence d'acquisition à l'extrémité 3' de ladite molécule miARN ; et
f) attacher à ladite molécule miARN un réactif d'acquisition comprenant un marquage capable de produire ou d'émettre un signal détectable et au moins une séquence d'acide nucléique complémentaire de ladite séquence d'acquisition,
en produisant de ce fait une molécule miARN cible marquée.

2. Procédé selon la revendication 1, dans lequel l'étape de liaison est effectuée en utilisant une ligase d'ADN T4.

3. Procédé selon la revendication 1, dans lequel le marquage comprend un fluorophore.

4. Procédé pour produire une molécule cADN marquée complémentaire d'une molécule mi ARN cible comprenant les étapes consistant à :
a) fournir une molécule miARN à brin unique ayant des extrémités 5' et 3' ;
b) attacher une queue d'oligonucléotides sur l'extrémité 3' de ladite molécule miARN à brin unique en utilisant une poly(A)polymérase ;
c) fusionner sur ladite queue d'oligonucléotides par pairage de base complémentaire une amorce à brin unique comprenant une séquence d'acquisition à son extrémité 5' et une séquence complémentaire à ladite queue d'oligonucléotides à son extrémité 3' ;
d) étendre ladite amorce à brin unique de son extrémité 3' avec une transcriptase inverse, en produisant de ce fait une molécule cADN à brin unique comprenant une séquence d'acquisition à son extrémité 5' ; et
e) attacher à ladite molécule cADN un réactif d'acquisition comprenant un marquage capable de produire ou d'émettre un signal détectable et au moins une séquence d'acide nucléique complémentaire de ladite séquence d'acquisition,
en produisant de ce fait une molécule cADN marquée complémentaire d'une molécule miARN cible.

5. Procédé selon la revendication 4, dans lequel l'amorce à brin unique est étendue avec une transcriptase inverse RNase H⁻.

6. Procédé selon la revendication 4, dans lequel le marquage comprend un fluorophore.

7. Procédé pour la détection d'une sonde d'anti-détection miARN sur un micro-réseau comprenant les étapes consistant à :
a) mettre en contact un micro-réseau sur lequel se trouve une sonde comprenant la séquence de nucléotides complémentaire d'une miARN avec une molécule miARN cible marquée produite par un procédé comprenant les étapes consistant à :
i) fournir une molécule miARN à brin unique ayant des extrémités 5' et 3' ;
ii) attacher une queue d'oligonucléotides sur l'extrémité 3' de ladite molécule miARN à brin unique en utilisant une poly(A)polymérase ;
iii) fournir une séquence d'acide nucléique partiellement à double brin ayant un brin de détection et un brin d'anti-détection, dans lequel le brin de détection comprend une séquence d'acquisition à son extrémité 3' et le brin d'anti-détection comprend une extrémité protubérante 3' à brin unique comprenant une séquence complémentaire de ladite queue d'oligonucléotides ;
iv) fusionner ladite séquence d'acide nucléique à double brin sur ladite queue d'oligonucléotides par pairage de base complémentaire avec la séquence d'extrémité protubérante 3' ;
v) lier l'extrémité 5' du brin de détection de ladite séquence d'acide nucléique partiellement à double brin à l'extrémité 3' de la queue d'oligonucléotides, en attachant de ce fait la séquence d'acquisition à l'extrémité 3' de ladite molécule miARN ; et
vi) attacher à ladite molécule miARN un réactif d'acquisition comprenant un marquage capable de produire ou d'émettre un signal détectable et au moins une séquence d'acide nucléique complémentaire de ladite séquence d'acquisition ;
en produisant de ce fait une molécule miARN cible marquée ;
b) incuber ledit micro-réseau et ladite molécule miARN cible marquée pendant un temps suffisant et à une température suffisante pour permettre à ladite molécule miARN cible marquée de s'hybrider sur ladite sonde d'anti-détection miARN ;
c) laver ledit micro-réseau pour enlever la miARN cible marquée déshybridée ; et
d) détecter le signal de la molécule miARN cible marquée hybridée,
en détectant de ce fait une sonde d'anti-détection miARN sur un micro-réseau.

8. Procédé selon la revendication 7, dans lequel la liaison est effectuée en utilisant une ligase d'ADN T4.

9. Procédé selon la revendication 7, dans lequel le marquage comprend un fluorophore.

10. Procédé selon la revendication 7, dans lequel le micro-réseau est incubé avec la molécule miARN cible à la suite de l'attachement du réactif d'acquisition.

11. Procédé selon la revendication 7, dans lequel le micro-réseau est incubé avec la molécule miARN cible avant l'attachement du réactif d'acquisition.

12. Procédé selon la revendication 7, dans lequel la sonde d'anti-détection miARN sur un micro-réseau est détectée par hybridation de molécules miARN cibles marquées produisant ou émettant au moins deux signaux détectables différents.

13. Procédé pour la détection d'une sonde de détection miARN sur un micro-réseau comprenant les étapes consistant à :
a) mettre en contact un micro-réseau sur lequel se trouve une sonde comprenant la séquence de nucléotides d'une miARN avec une molécule cADN marquée complémentaire d'une molécule miARN cible produite par un procédé comprenant les étapes consistant à :
i) fournir une molécule miARN à brin unique ayant des extrémités 5' et 3' ;
ii) attacher une queue d'oligonucléotides sur l'extrémité 3' de ladite molécule miARN à brin unique en utilisant une poly(A)polymérase ;
iii) fusionner sur ladite queue d'oligonucléotides par pairage de base une amorce à brin unique comprenant une séquence d'acquisition à son extrémité 5' et une séquence complémentaire à ladite queue d'oligonucléotides à son extrémité 3' ;
iv) étendre ladite amorce à brin unique de son extrémité 3' avec une transcriptase inverse, en produisant de ce fait une molécule cADN à brin unique comprenant une séquence d'acquisition à son extrémité 5' ; et
v) attacher à ladite molécule cADN un réactif d'acquisition comprenant un marquage capable de produire ou d'émettre un signal détectable et au moins une séquence d'acide nucléique complémentaire de ladite séquence d'acquisition,
en produisant de ce fait une molécule cADN marquée complémentaire d'une molécule miARN cible ;
b) incuber ledit micro-réseau est ladite molécule cADN marquée pendant un temps suffisant et à une température suffisante pour permettre à ladite molécule cADN marquée de s'hybrider sur ladite sonde d'anti-détection miARN ;
c) laver ledit micro-réseau pour enlever la cADN marquée déshybridée ; et
d) détecter le signal de la molécule cADN marquée hybridée,
en détectant de ce fait une sonde d'anti-détection miARN sur un micro-réseau.

14. Procédé selon la revendication 13, dans lequel l'amorce à brin unique est étendue avec une transcriptase inverse RNase H⁻.

15. Procédé selon la revendication 13, dans lequel le marquage comprend un fluorophore.

16. Procédé selon la revendication 13, dans lequel le micro-réseau est incubé avec la molécule cADN à la suite de l'attachement du réactif d'acquisition.

17. Procédé selon la revendication 13, dans lequel le micro-réseau est incubé avec la molécule cADN avant l'attachement du réactif d'acquisition.

18. Procédé selon la revendication 13, dans lequel la sonde de détection miARN sur un micro-réseau est détectée par hybridation de molécules cADN marquées complémentaires des molécules miARN cibles produisant ou émettant au moins deux signaux détectables différents.
